**Europäisches Patentamt**

**European Patent Office**

**Office européen des brevets**

(11) Publication number : **0 354 315 B1**

# EUROPEAN PATENT SPECIFICATION

(45) Date of publication of patent specification :
**13.05.92 Bulletin 92/20**

(21) Application number : **89110316.0**

(22) Date of filing : **07.06.89**

(51) Int. Cl.⁵ : **A61F 13/02**

(54) **Adhesive dressing sheet.**

(30) Priority : **11.08.88 JP 106557/88 U**

(43) Date of publication of application :
**14.02.90 Bulletin 90/07**

(45) Publication of the grant of the patent :
**13.05.92 Bulletin 92/20**

(84) Designated Contracting States :
**DE FR GB SE**

(56) References cited :
**EP-A- 0 081 989**
**EP-A- 0 144 891**
**EP-A- 0 189 999**

(73) Proprietor : **NITTO DENKO CORPORATION**
**1-2, Shimohozumi 1-chome Ibaraki-shi**
**Osaka (JP)**

(72) Inventor : **Sugii, Tetsuji c/o NITTO DENKO**
**CORPORATION**
**1-2, Shimohozumi 1-chome**
**Ibaraki-shi Osaka (JP)**
Inventor : **Wada, Shintaro c/o NITTO DENKO**
**CORPORATION**
**1-2, Shimohozumi 1-chome**
**Ibaraki-shi Osaka (JP)**
Inventor : **Konno, Masayuki c/o NITTO DENKO**
**CORPORATION**
**1-2, Shimohozumi 1-chome**
**Ibaraki-shi Osaka (JP)**

(74) Representative : **Patentanwälte Grünecker,**
**Kinkeldey, Stockmair & Partner**
**Maximilianstrasse 58**
**W-8000 München 22 (DE)**

## Description

### FIELD OF THE INVENTION

The present invention relates to an adhesive dressing sheet which is used for covering of a cut, fixation of an IV catheter, and so forth.

### BACKGROUND OF THE INVENTION

An adhesive dressing sheet usually has a structure that a polymer film having excellent skin fit properties is used as a substrate, an adhesive layer is provided on one side of the substrate and a peeling liner is temporarily provided on the adhesive layer as disclosed in, for example, JP-A-U-57-123129 and 58-124123. The term "JP-A-U" as used herein means an "unexamined published Japanese utility model application". In such adhesive dressing sheets, it is important that the sheet is accurately adhered to the skin while examining the skin portion to be covered. For this reason, it is considered that a bending line is previously provided on the peeling liner, and while bending the liner at the bending line and utilizing the bending state, the sheet is bonded to the skin while examining the affected part of the skin to be covered.

However, a polymer film 1' which is used as the substrate as shown in Fig. 7 is a thin film-like material having a low modulus from a standpoint of skin adhesion properties and therefore does not have an elasticity against bending force. Thus, as shown in Fig. 7, when a peeling liner 3' is peeled off up to a bending line 32', liner bending pieces 30', 30' expand, and only by application of a slight tensile stress, the liner remaining piece is inevitably continuously peeled off from a surface of an adhesive layer 2'. Therefore, careful handling is required and it is not easy to adhere the sheet while examining the affected part of the skin to be covered.

A dressing sheet in which a backing sheet is temporarily bonded to the back side of the polymer film 1' so that the polymer film 1' does not wrinkle at the time of bonding is also proposed as disclosed in, for example, JP-A-U-62-42814. In such dressing sheets, however, it is sufficient for the backing sheet to have supporting properties to the extent that the backing sheet bonds to the polymer film 1' and does not wrinkle and, therefore, the above problem due to insufficient bending elasticity as encountered in providing a bending line on the peeling liner and bending the liner cannot be avoided.

### SUMMARY OF THE INVENTION

Accordingly, an object of the present invention is to provide a novel adhesive dressing sheet which can overcome the above-described problems.

The present invention provides an adhesive dressing sheet having a structure that an adhesive layer is provided on one side of a polymer film having a high flexibility and a peeling liner is provided on the adhesive layer, and on the other side of the polymer film, a flexible sheet is temporarily provided, wherein a bending line is provided on the peeling liner in a direction substantially at right angles to the liner peeling direction, and the flexible sheet has self supporting properties such that when the liner is peeled off up to the bending line and bent, the bent part of the liner does not bend through an angle of 90° or more.

### BRIEF DESCRIPTION OF THE DRAWINGS

Figure 1A illustrates an example of the present invention and is a plan view seen from the side of the peeling liner;
Figure 1B is a cross sectional view taken in the plane of line I-I' in Fig. 1A;
Figures 2A and 2B are views illustrating the manner of use thereof;
Figure 3 is a cross sectional view illustrating the state that the peeling liner is peeled off;
Figure 4A illustrates another example of the present invention and is a plan view seen from the side of the flexible sheet;
Figure 4B is a cross sectional view taken in the plane of line I-I' in Figure 4A;
Figure 5 is a cross sectional view illustrating another example of the present invention;
Figure 6 is a cross sectional view illustrating the peeling liner-peeled off state in a case where a cutting line is provided on the flexible sheet at a position at which it overlaps with the bending line of the peeling liner; and
Figure 7 is a cross sectional view of the conventional example illustrating the state that the peeling liner is peeled off.

In the drawings, 1 indicates a polymer film; 2, an adhesive layer; 3, a peeling liner; 32, a bending line; and 4, a flexible sheet.

### DETAILED DESCRIPTION OF THE INVENTION

The present invention is now explained in detail by reference to the drawings.

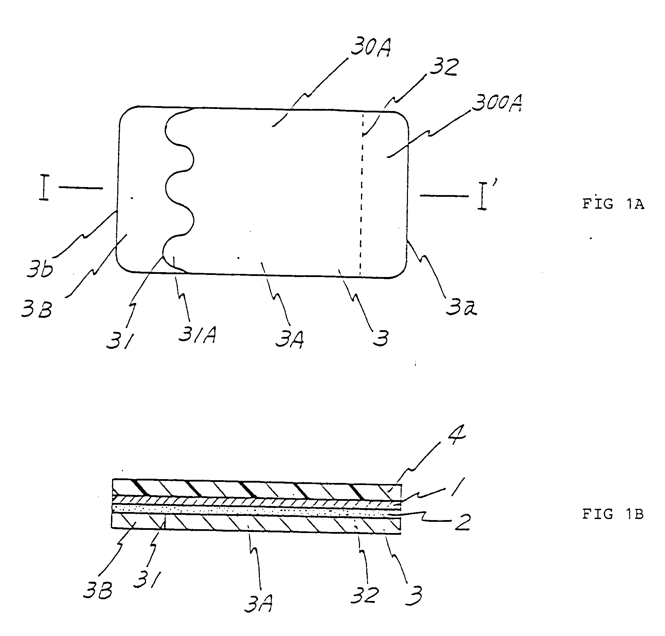

In Figures 1A and 1B, 1 indicates a polymer film which is obtained by molding a polyetherurethane, a polyesterurethane, an acryl polymer, a nylon derivative, elastomeric polyester, a mixture of polyester and polyurethane, or the like into a film having a thickness of about 20 to 150 µm and has such a high flexibility that 50% modulus is about 20 to 200 kg/cm$^2$. The film 1 desirably has a moisture permeability so as not prevent the respiration of the skin after adhering the film to the skin. 2 indicates an adhesive layer of e.g., acryl, which is provided on one side of the film 1 and, if necessary, a desired drug is contained in the adhesive layer. 3 indicates a peeling liner temporarily provided on the layer 2, and for example, a peeling paper subjected to silicone treatment and having a weight of about 80 to 200 g/m$^2$ is used. In the peeling liner 3, a cutting line 31 (e.g. cut) in the wave form extending along the end 3b and in the edge direction of the edge 3b is provided, and the liner 3 is divided into divided pieces 3A, 3B by the cutting line 31. In the larger divided piece 3A, a straight bending line 32 (e.g. perforation) is provided along the other end 3a and in a direction almost in parallel to the above cutting line 31, and the divided piece 3A is partitioned into a central portion 30A having a relatively large area and a side portion 300A. The direction in which the bending line 32 is provided is almost at right angles to the peeling direction (f) of the liner divided piece 3A. 4 indicates a flexible sheet fitted to the other side of the film 1. The sheet 4 is made of a material having a thickness of about 25 to 75 $\mu$m, and selected from plastic sheets, paper and so forth, so as to have self supporting properties such that when the liner divided piece 3A is peeled off up to the bending line 32 and bent, the liner bended part (divided piece 3A) does not expand to the angle of 90° or more.

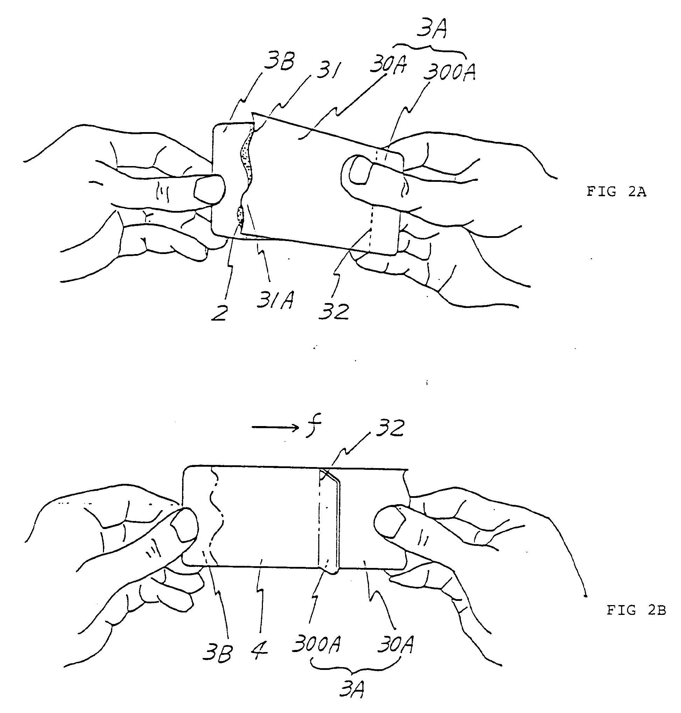

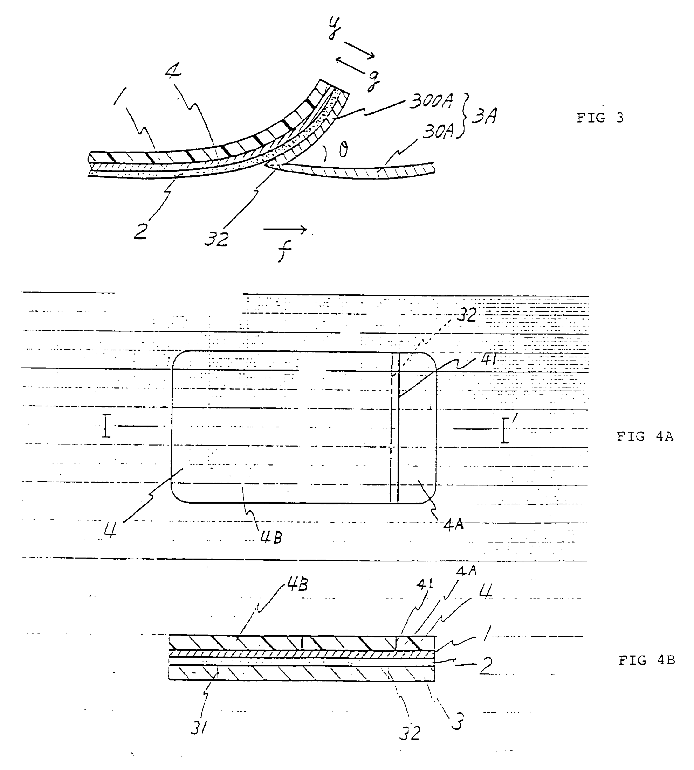

In the adhesive dressing sheet having the above-described structure, as illustrated in Fig. 2A, when the whole sheet is bent to the side of the flexible sheet 4, the peeling liner 3 is divided into the divided pieces 3A and 3B by the cutting line 31 portion. The smaller divided piece 3B portion of the divided pieces is held with fingers of one hand, and at the same time, the cut convex portion 31A of the larger divided piece 3A is held by fingers of the other hand and picked up to peel off from the sheet. When the larger divided piece 3A is peeled off up to the bending line 32, the divided piece 3A is bent at this part by the line 32. At this time, the great self supporting properties of the flexible sheet 4 exhibit repelling elasticity (y) against recovering force (g) of the remaining liner pieces (side portion 300A of divided piece 3A) and prevent the bent divided piece 3A from expanding to an angle θ=90° or more. Thus, as illustrated in Figure 3, even if a tensile stress is applied to the central portion 30A constituting the divided piece 3A, a stress acts on the side portion 300A not in the cleavage direction but in the shear direction and, therefore, the side portion 300A is not easily peeled off from the adhesive surface and peeling of the portion is temporarily stopped. Then, while examining the skin surface on which the sheet is bonded, the sheet central portion is bonded thereto at the adhesive layer 2 surface and, thereafter, the divided piece 3A is strongly pulled out. In this manner, the liner residual piece (side portion 300A) is removed from the layer 2 surface, and the entire surface of the layer 2 is attached to the skin. The flexible sheet 4 which is made into one unit along with the polymer film 1 is peeled off and removed from the surface of the film 1, and adhereing is completed without touching of fingers to the adhesive surface.

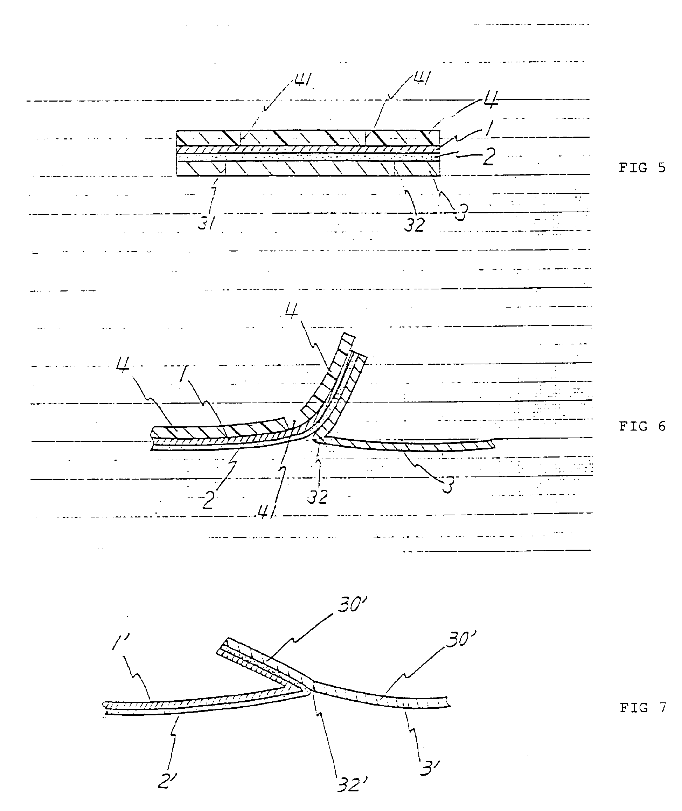

Figures 4A, 4B and 5 illustrate other examples of the present invention. In Figures 4A and 4B, the cutting line 41 is provided in a direction in parallel to the bending line 32 on the flexible sheet 4 so as to divide the sheet into two portions 4A, 4B, and in Figure 5, cutting lines 41 and 41 are provided so as to divide the flexible sheet 4 into three portions. If cutting lines are provided on the flexible sheet 4, when the whole sheet is attached to the skin surface, the sheet is bent according to the raised form of the skin surface, and there is formed a condition that the divided pieces 4A, 4B of the sheet 4 come to the surface from the cut line portion. Therefore, peeling and removal of the sheet becomes easy. For this reason, when a cutting line is provided on the flexible sheet 4, it is necessary for the cutting line to be provided at a location at which the line crosses with the bending line 32 of the liner 3, or at a location at which it is, as illustrated in the Figures 4A, 4B and 5, in a parallel condition and does overlap. If the cutting line overlaps with the bending line of the liner, the desired effects of the present invention are not exhibited. That is, the bending stress when the liner is bent at the bending line is concentrated at the bending line portion in the case of the flexible sheet 4. In the present invention, since the sheet 4 exhibits, as described above, great repression elasticity, the bending stress is rather concentrated at the cutting line portion and the sheet 4 is easily peeled off before attaching (Figure 6) and, therefore, handling becomes difficult.

In the present invention, in order to facilitate that a position to be adhered can be easily confirmed in the adhering operation, it is desirable that polymer film 1, the adhesive layer 2 and the flexible sheet 4 be transparent.

In the present invention, peeling of the liner bent piece can be temporarily stopped by bending the liner at the bending line provided on the peeling liner. Therefore, even if a tensile stress is applied, the remaining piece of the liner is not continuously peeled off, the sheet can be accurately bonded by fingers while confirming the skin surface to be bonded, and the handling is easy.

While the invention has been described in detail and with reference to specific embodiments thereof, it will be apparent to one skilled in the art that various changes and modifications can be made therein without departing from the scope thereof as defined in the claims.

**Claims**

1. An adhesive dressing sheet comprising a polymer film (1) having a high flexibility, an adhesive layer (2) provided on one side of the polymer film, a peeling liner (3) temporarily provided on the adhesive layer, and a flexible sheet (4) on the other side of the polymer film, wherein a bending line (32) is provided on the peeling liner in a direction substantially at right angles to the peeling direction of the liner, characterised in that the flexible sheet (4) has self supporting properties such that when the liner is peeled off up to the bending line and bent, the bent part of the liner does not bend through an angle of 90° or more.

2. The sheet as claimed in Claim 1, wherein a cutting line (31) is provided on the peeling liner so as to divide the liner.

3. The sheet as claimed in Claim 1, wherein a cutting line (41) is provided on the flexible sheet in such a manner that the cutting line does not overlap the bending line of the peeling liner.

4. The sheet as claimed in Claim 1, wherein the polymer film, the adhesive layer and the flexible sheet are transparent.


## Patentansprüche

1. Haft-Verbandsblatt, umfassend einen Polymerfilm (1) mit hoher Flexibilität, eine Klebeschicht (2), die auf einer Seite des Polymerfilms vorgesehen ist, eine Abzieheinlage (3), die vorübergehend auf der Klebeschicht vorgesehen ist, und ein flexibles Blatt (4) auf der anderen Seite des Polymerfilms, wobei eine Biegelinie (32) auf der Abzieheinlage in einer zur Abziehrichtung der Einlage im wesentlichen rechtwinkligen Richtung vorgesehen ist, dadurch gekennzeichnet, daß das flexible Blatt (4) selbsttragende Eigenschaften besitzt, so daß, wenn die Einlage in Richtung der Biegelinie abgezogen und gebogen wird, der gebogene Teil der Einlage sich nicht über einen Winkel von 90° oder mehr biegt.

2. Blatt nach Anspruch 1, dadurch gekennzeichnet, daß die Schnittlinie (31) auf der Abzieheinlage vorgesehen ist, um so die Einlage zu teilen.

3. Blatt nach Anspruch 1, dadurch gekennzeichnet, daß eine Schnittlinie (41) auf dem flexiblen Blatt in der Weise vorgesehen ist, daß die Schnittlinie nicht die Biegelinie der Abzieheinlage überlappt.

4. Blatt nach Anspruch 1, dadurch gekennzeichent, daß der Polymerfilm, die Klebeschicht und das flexible Blatt transparent sind.


## Revendications

1. Feuille de pansement adhésive comportant un film polymère (1) présentant une grande flexibilité, une couche adhésive (2) prévue sur l'une des faces du film polymère, une couche de protection pelable (3) temporairement prévue sur la couche adhésive, et une feuille flexible (4) prévue sur l'autre face du film polymère, étant précisé qu'une ligne de pliage (32) est prévue sur la couche de protection pelable selon une direction substantiellement à angle droit par rapport à la direction selon laquelle se pèle la couche de protection, feuille de pansement caractérisée par le fait que la feuille flexible (4) présente les caractéristiques d'auto-support telles que, lorsque l'on enlève la couche de protection, en la pelant, jusqu'à la ligne de pliage et qu'on la plie, la partie pliée de la couche de protection ne se plie pas sur un angle de 90° ou davantage.

2. Feuille de pansement comme revendiquée dans la revendication 1, dans laquelle une ligne de coupe (31) est prévue sur la couche de protection pelable de façon à diviser cette couche de protection.

3. Feuille de pansement comme revendiquée dans la revendication 1, dans laquelle une ligne de coupe (41) est prévue sur la feuille flexible de façon que cette ligne de coupe ne coincide pas avec la ligne de pliage de la couche de protection pelable.

4. Feuille de pansement comme revendiquée dans la revendication 1, dans laquelle le film polymère, la couche adhésive et la feuille flexible sont transparents.

FIG 1A

FIG 1B

FIG 2A

FIG 2B

FIG 3

FIG 4A

FIG 4B

FIG 5

FIG 6

FIG 7